Europäisches Patentamt

European Patent Office

Office européen des brevets

(11) Publication number: **0 531 728 A1**

# EUROPEAN PATENT APPLICATION

(21) Application number: **92113634.7**

(22) Date of filing: **10.08.92**

(51) Int. Cl.⁵: **C12N 15/38**, C12N 15/86, C12N 5/10, A61K 39/245, C07K 13/00

(30) Priority: **13.09.91 US 759310**

(43) Date of publication of application:
**17.03.93 Bulletin 93/11**

(84) Designated Contracting States:
**AT BE CH DE DK ES FR GB GR IE IT LI LU NL PT SE**

(71) Applicant: **AMERICAN CYANAMID COMPANY**
**1937 West Main Street P.O. Box 60**
**Stamford Connecticut 06904-0060(US)**

(72) Inventor: **Landolfi, Victoria Ann**
**36 Darin Road**
**Warwick, New York 10990(US)**
Inventor: **Figueroa, Natalie Beth**
**40 Morningside Drive**
**Patterson, New York 12563(US)**

(74) Representative: **Wächtershäuser, Günter, Dr.**
**Tal 29**
**W-8000 München 2 (DE)**

(54) HSV gD2 Baculovirus expression system.

(57) The present invention relates to a method of production of recombinant HSV2 gD (gD2) in a baculovirus expression system, and the recombinant glycoprotein produced thereby. The glycoprotein produced is highly immunogenic and is useful in vaccine preparations for the prevention and treatment of HSV infections.

EP 0 531 728 A1

## FIELD OF THE INVENTION

The present invention relates to recombinantly produced viral proteins. More specifically, the invention relates to the recombinant production of Herpes Simplex Virus type 2 (HSV2) glycoprotein D (gD2) by baculovirus in an insect host cell. The gD2 produced thereby is immunogenic and protective, and as such is useful in a vaccine composition to protect against HSV infection.

## BACKGROUND OF THE INVENTION

Infection with herpes simplex virus (HSV) types 1 and 2 remains a prevalent and potentially serious health problem, particularly in neonates and in immunocompromised individuals. In addition to mucocutaneous surfaces, virus infection may involve the eyes, brain or other organs and has been implicated as a potential causative agent of cervical cancer (2,24). In addition, HSV establishes latent infections with the potential for recurrent episodes of disease (30) with attendant physical and psychological discomfort. The development of an HSV vaccine has been hampered by the association of the viral genome with oncogenesis (3) and has directed the focus of vaccine efforts towards subunit approaches to immunoprophylaxis and immunotherapy.

Herpes simplex virus glycoprotein D (gD) is a primary vaccine candidate for use in humans. Associated both with the virus envelope and the infected cell surface, gD has been shown to play an indispensable role in the early events of virus infection (11, 9) and to serve as an important target of cross-reactive neutralizing antibody (4) and cellular immune responses (1, 22, 40, 47, 11, 35, 31). It has been previously shown that a native preparation of gD, derived from HSV1-infected Vero cells, confers protection against HSV2 challenge in mice and in guinea pigs, elicits long-term humoral and cellular responses in primates (25) and is safe upon administration to healthy seropositive adults (9). To optimize yields of glycoprotein and to obviate potential introduction of virus or reported transforming regions of the genome in vaccine inocula, a number of recombinant systems have been used in the production of gD. These include bacterial (44), yeast (37, 41), vaccinia (28), mammalian (36) and baculovirus (14) expression systems. Products of these systems have shown varying degrees of immunogenicity in animal models. A baculovirus system offers advantages of safety, acceptable fidelity of posttranslational modification (13, 15), immunogenicity (33, 42, 14, 34, 8) as well as the potential for substantial yields of foreign protein (45, 6) when placed under the control of the baculovirus polyhedrin promoter.

The present invention provides for the first time a baculovirus-expressed herpes simplex virus type 2 glycoprotein D (bgD2) using Spodoptera frugiperda cells as the substrate for infection. Resultant glycoprotein is purified and shown to conform to the biochemical and immunochemical characteristics of HSV-derived gD. In addition, mice immunized with this vaccine candidate illustrate humoral and cellular responses to whole virus and purified glycoproteins and are protected against lethal HSV2 challenge.

## SUMMARY OF THE INVENTION

The present invention relates to a method of producing recombinant gD2 comprising transfecting insect host cells with baculovirus DNA incorporating the gene for gD2, and culturing the cells under conditions that permit expression of the gene. In a preferred embodiment, the gD2 gene is placed under control of the baculovirus polyhedrin promoter. The glycoprotein produced thereby is shown to be highly immunogenic and protective against challenge with lethal doses of HSV. The invention thus also provides vaccine compositions comprising an effective amount of a substantially pure baculovirus-produced gD2, in combination with a pharmaceutically acceptable carrier. Such compositions can be used in a method of treatment or prevention of HSV infection comprising administering to a host an effective amount of the vaccine composition.

Also provided by the present invention are baculovirus vectors useful in expression of the gD2 gene in insect cells, as well as the insect cells infected thereby.

## BRIEF DESCRIPTION OF THE FIGURES

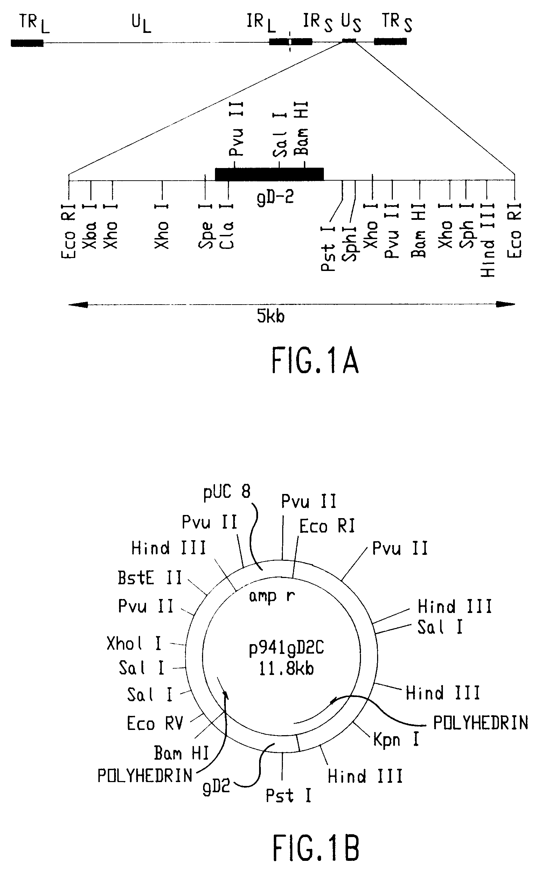

**Figure 1.** Location of the gD2 gene in the HSV2 genome and its placement in the baculovirus transfer vector. (A) The herpes simplex virus type 2 (strain 12) genome (43) is digested with Eco RI to isolate the gD2 gene on a 5 kb fragment. (B) An Spe I/Xho I fragment containing gD2 is inserted into the unique BamHI site of the baculovirus transfer vector pVL941 by blunt end ligation. The Spe I and Xho I sites at the 5' and 3' ends of the insert, respectively, are lost after the blunt end ligation. However, the BamHI site is retained.

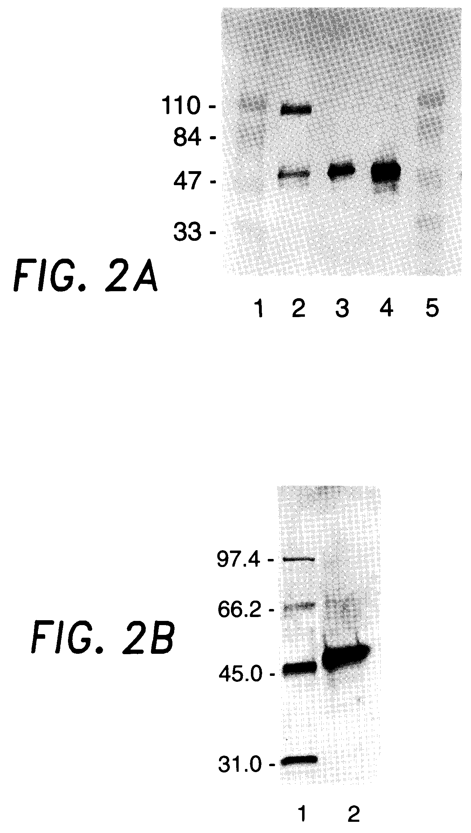

**Figure 2.** SDS-PAGE analysis of ngD1, ngD2 and bgD2. The purified proteins are subjected to SDS-PAGE electrophoresis and electroblotted onto nitrocellulose. FRAME A: lane 1, molecular weight standards; lane 2, purified native gD1; lane 3,purified native gD2; lane 4, purified recombinant gD2. The blot was immunoprobed with rabbit polyclonal anti-gD serum and visualized with peroxidase labeled protein A and 4 chloronaphthol/$H_2O_2$. FRAME B: lane 1, molecular weight standards; lane 2, purified recombinant gD2. Protein bands on the blot are visualized by silver staining.

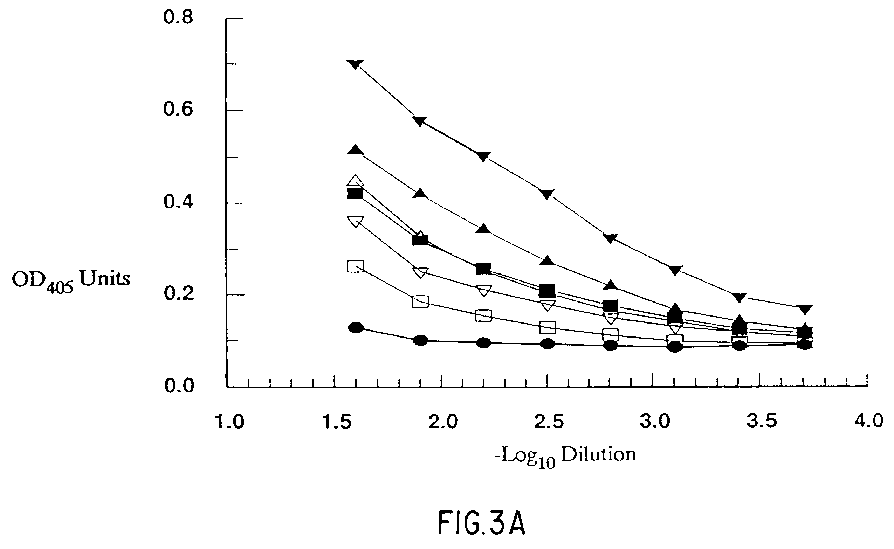

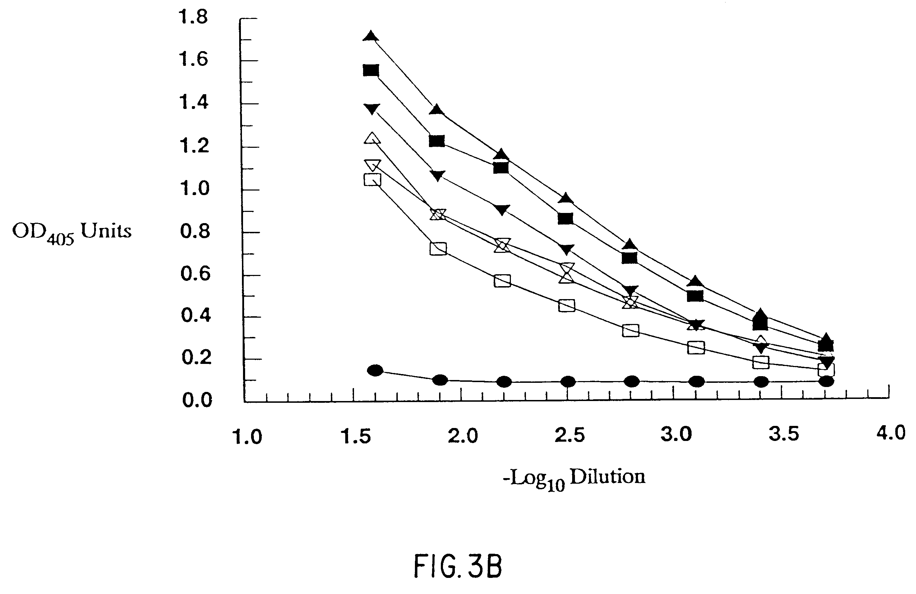

**Figure 3.** ELISA responses to baculovirus-expressed and native gD. Mean ELISA responses of 5 mice per group to ngD1 (Figure 3A) and baculovirus-expressed gD2 (Figure 3B) antigens. General patterns of bgD dose-related increases in activity were noted for both antigens. Immunizations were $AlPO_4$ (-o-); 0.1 $\mu$g bgD2 (-⊞-); 1.0 $\mu$g bgD2 (-■-); 5.0 $\mu$g bgD2 (-△-); 10.0 $\mu$g bgD2 (-▲-); 5.0 $\mu$g ngD2 (-▽-); 5.0 $\mu$g ngD1 (-▼-).

## DETAILED DESCRIPTION OF THE INVENTION

The present method employs a baculovirus expression system for high-yield production of gD2 which closely approximates the structure of the native gD2 glycoprotein (ngD2). A number of baculoviruses, in particular the nuclear polyhedrosis viruses (NPV) are propagatable in insect cell culture, and provide a potentially useful vehicle for glycoprotein production. Useful NPVs include the Bombyx mori NPV (BmNPV; 19) and Autographa californica NPV (AcNPV; 39). A preferred baculovirus for use in the present method is the AcNPV. To initiate the procedure, the DNA fragment containing the gD2 sequence is isolated and subcloned into a baculovirus transfer vector. The selected insect cells are then cotransfected with the transfer vector and AcNPV DNA, so that the gene for gD2 is inserted into the baculovirus genome, replacing the virus' polyhedrin gene, and under the control and regulation of the polyhedrin promoter. There are a number of transfer vectors available for this purpose (e.g., 18, 23). A preferred vector is one which gives high levels of expression with gD2, but which does not produce a fusion protein with polyhedrin. In the present examples such a vector, pVL941 (18), is used. It is generally believed that sequences immediately 5' to and possibly extending downstream from the polyhedrin start codon are important for transcription. In pVL941, the gD2 gene is inserted downstream of the ATG start site of the polyhedrin gene, and the leader sequence is intact. To eliminate the fusion protein, the ATG start site of the polyhedrin gene is altered to ATT by site-directed mutagenesis.

A number of insect cell lines can be used as host cells in the present method. For example, cells of the species Mamestra brassicae (such as ATCC CRL 8003) or Spodeptera frugiperda (such as Sf-21, available from Invitrogen, San Diego, CA or Sf-9, ATCC CRL 1711) are useful in the present method. A preferred host cell line is Spodontera frugiperda. Recombinant baculovirus plaques are identified and picked by the absence of polyhedrin occlusion bodies. These plaques are amplified on insect cells and resultant plaques screened for baculovirus gD2 (bgD2) expression. Selected plaques can be purified and used for further gD2 expression.

The gene used for expression can be any HSV gene possessing the epitopes required for immunogenicity and protection. Sequence of gD2 from HSV strain G have been published by Watson (43) and Lasky, et al. (16). In the following examples, HSV2 strain 12 is used as the source for gD2 DNA. The DNA sequence of these two genes differs to a minor extent, ultimately producing a difference of one amino acid in the resulting protein (see Example 3). It will be seen that minor modifications in the basic gD2 sequence can be tolerated without affecting the immunogenicity of the molecule. Therefore, the gene used in the process can also be one which differs from known sequences at the nucleotide level, but which, because of the degeneracy in the genetic code, produce the same amino acid sequence, or which produces "silent changes" in which one chemically equivalent amino acid is substituted for another without affecting the immunogenic activity of the resulting glycoprotein.

The present method yields a glycoprotein with an apparent molecular weight of 57,500 daltons, which is approximately the same as ngD2. The glycoprotein is recovered from disrupted cells and purified by immunoaffinity chromatography, which provides purification of at least about 90%. The identity of the protein is confirmed by Western blot using gD2-specific monoclonal and polyclonal antibodies. The nonglycosylated weight of the protein predicted from the amino acid sequence is about 40,000, suggesting extensive posttranslational addition of carbohydrate. Glycosylation patterns of proteins expressed in baculovirus infected insect cells have been demonstrated to be tunicamycin-(13, 14) and endo-H (45, 15) sensitive suggesting the presence of N-linked high mannose glycosylation patterns. As shown in the Examples below, ELISA and immunoblotting reactivities of bgD2 appear to surpass those of native preparations in binding of poly- and monoclonal antibodies. These observations suggest a potentially enhanced accessibility of epitopes in the baculovirus-expressed glycoprotein. The augmented serological responsiveness to baculovirus-expressed gD2 may indicate an enhanced potency of the recombinant product relative to the native material with respect to antibody responsiveness.

The purified glycoprotein reacted in Western or dot blots with the monoclonal antibodies DL6, DL11 and 1D3. DL11 binds a nonsequential neutralizing epitope (29) of viral-derived gD. Thus, it is concluded that the recombinant vaccine preparation retains native conformation at this site. Indeed, the maintenance of native conformation has been linked with DL11 reactivity and its complement independent neutralizing capability (27, 46). Reactivity with 1D3 MAb suggests that the glycoprotein is not truncated at the amino terminus.

Similarity of the baculovirus-derived molecule to the native form was confirmed by serological studies. ELISA analysis of serum from immunized mice showed that ngD1 immune serum reacts strongly with bgD2. Conversely bgD2-immune sera bind both ngD1 and bgD2, even after a vaccine dose as low as 0.1 μg. ELISA titers are observed to increase with bgD2 dose. Neutralizing antibody titers of bgD2 immunized mice to HSV1 and HSV2 increase in a generally dose-related manner and demonstrated preferentially elevated titers to homotypic virus although geometric mean titers against the heterotype were substantial at doses greater than 0.1 μg bgD2. A similar pattern of higher titer against HSV2 is noted for sera from ngD2 immunized mice suggesting that this phenomenon is not unique to immunization with recombinant glycoprotein. Baculovirus expressed gD2 elicits enhanced levels of neutralizing antibody against HSV2 when compared with both ngD1 and ngD2. Sera from ngD1 immunized mice also demonstrate elevated titers to HSV2 when compared with those for HSV1. However, the difference observed for ngD1 is modest.

When bgD2 immunization is compared with ngD1 and ngD2 vaccine with respect to in vitro lymphoproliferative responses to whole viral and subunit antigens, bgD2 in vitro cellular responses are characterized by an essentially type-specific response to whole viral antigen and by an inverse correlation between bgD2 dose and lymphoproliferative activity. The observed associations between vaccine concentration, increased antibody responsiveness, and reduced lymphoproliferative activity suggest a possible gD dose-related selection of T helper cell subsets (26).

In vivo, immunization with bgD2 results in significant protection against lethal HSV2 footpad challenge. High levels of protection occur at all doses of glycoprotein administered. Protection against HSV2 is characterized by reduced severity of symptoms including paralysis and death and by significant increases in the frequency of mice free of all observable symptoms of infection. While the frequency of latent HSV2 infection in surviving mice was not determined in the present study, we have previously shown that immunization with ngD1 in mice protects against latent as well as primary HSV2 infection (25).

The foregoing information clearly shows the utility of a baculovirus expressed gD2 in a vaccine composition. The glycoprotein, or an immunogenic portion thereof, in substantially pure (i.e., at least about 90% pure form can be used in both prevention and treatment of HSV1 and HSV2 infections.

The recombinantly produced glycoprotein is isolated from host cells by standard protein isolation techniques. The purified glycoprotein is then combined with any of the commonly used pharmaceutically acceptable carriers, such as water, physiological saline, ethanol, polyols, such as glycerol or propylene glycol, or vegetable oils, as well as any appropriate vaccine adjuvants, for example, AlPO$_4$, and/or 3 dioxy-monophosphonyl lipid A (3D-MPL; RIBI). As used herein, "pharmaceutically acceptable carriers" encompasses any and all solvents, dispersion media, coatings, antifungal agents, isotonic and absorption delaying agents and the like. Except insofar as any conventional medium is incompatible with the active ingredient, its use in the vaccine composition is contemplated.

In addition to its use a sole immunogenic agent in a vaccine, the bgD2 may also be combined with other active agents. For example, an HSV vaccine may comprise additionally other HSV glycoproteins, such as gB1 and/or gB2, or with immunogenic agents against other infectious diseases, such as hepatitis or influenza.

The vaccine compositions are formulated with an amount of the bgD2 effective to confer protection against a subsequent exposure to HSV infection. Methods of formulation of vaccine compositions is known in the art, as shown for example in Duffy (6) or Williams et al. (47). The amount of glycoprotein used will vary, depending on whether an adjuvant is employed. Generally, a unit dose of vaccine will contain from 1 to 100 μg of glycoprotein. Administration is preferably parenteral, i.e., subcutaneous, intravenous, intramuscular or intraperitoneal, but may alternately be given orally.

The initial vaccination will preferably be followed by one or more boosts.

The microorganisms referred to herein are retained in the collection of American Cyanamid Company, Lederle Laboratories, Pearl River, New York. Also, plasmid p941gD2C has been deposited under the terms of the Budapest Treaty with the American Type Culture Collection, 12301 Parklawn Drive, Rockville, Maryland, on August 9, 1991, as Accession No. ATCC 68664.

The following non-limiting examples further illustrate the present invention.

4

## EXAMPLES

### I. Baculovirus Expression of gD2

**A. Cells and Virus.** Wild type Autographa californica nuclear polyhedrosis virus (AcNPV; E2 strain ATCC VR 1344) and baculovirus transfer vector pVL941 are received from Dr. Max summers (Texas A&M, College Station, TX). Spodoptera frugiperda (Sf9) cells are purchased from the American Type Culture Collection (ATCC CRL 1711; Rockville, MD). Wild type and recombinant baculovirus are grown in monolayer or suspension cultures of Sf9 cells in IPL41 medium (JRH Biosciences, Lenexa, KS) containing 2% iron supplemented calf serum (Hyclone, Logan, UT), 0.1% Pluronic F-68, and other lipid additions (17). Herpes simplex virus type 2 (strain 12; HSV2-12) and HSV1-NS are received from Dr. H. Friedman (Children's Hospital, Philadelphia PA) and are plaque purified 3 times and propagated in Vero cell culture. HSV2-186 is subjected to similar plaque purification and Vero cell propagation procedures, for use in animal studies.

**B. Construction of Transfer Vectors.** The glycoprotein D of HSV2 (strain 12) is expressed in S. frugiperda cells (Sf9) by the recombinant baculovirus D2AC-11. D2Ac-11 is generated by replacing the polyhedrin gene of Autographa californica nuclear polyhedrosis virus with the gD2 gene under the control and regulation of the polyhedrin promoter. HSV2 DNA is isolated from 2 liters of an HSV2 infected Vero cell lysate by passing through a DEAE Sepharose column. The DNA is eluted from the column with 0.5 M NaCl and treated with 40 $\mu$g/ml RNAse A, 100 $\mu$g/ml Proteinase K, and 0.4% SDS followed by several extractions with phenol/chloroform/isoamyl alcohol mixture (25:24:1). DNA is then purified on a CsCl gradient at a density of 1.3 g/ml. The purified DNA is digested with EcoRI and probed for the HSV2 glycoprotein D (gD2) gene using an RNA transcript of glycoprotein D from HSV1 (gD1). The plasmid pRE26 (received from Drs. G.H. Cohen and R.J. Eisenberg, University of Pennsylvania, Philadelphia PA), which contains the HSV1 gD (gD1) gene under control of the SP6 promoter is used. A $^{32}$P-CTP (410 Ci/MMol, Amersham, Arlington Heights, IL) labeled RNA transcript is prepared using SP6 polymerase. The gD2 gene is located on an Eco RI fragment, as demonstrated by Southern blot hybridization. The 5 kb Eco RI fragment (Figure 1A) is agarose gel purified and inserted into the unique Eco RI site of pBR322 using cloning procedures according to Maniatis et al. (21) to generate the plasmid p322gD2. This construct is double digested with Spe I and Xho I to trim the insert down to approximately 30 bases upstream from the gD2 ATG start site. The resultant 2 kb gD2-containing fragment is agarose gel purified and subcloned into the unique BamHI site of the transfer vector pVL941 (17) by blunt end ligation. The orientation of the resulting plasmid p941gD2C (Figure 1B) is confirmed by restriction mapping, Southern blot hybridization, and DNA sequencing by the dideoxy method using Sequenase, a T7 DNA polymerase (US Biochemical, Cleveland, OH).

**C. Generation of a Baculovirus Recombinant.** Sf9 cells are co-transfected with 2.0 $\mu$g of AcNPV DNA and 4.0 $\mu$g of p941gD2C DNA according to the procedure of Summers and Smith (38). Recombinant baculovirus plaques are identified microscopically by the absence of polyhedrin occlusion bodies. Occlusion body negative plaques are picked and amplified on Sf9 cells seeded in 24 well plates. Resultant plaques are then screened for baculovirus gD2 (bgD2) expression by SDS-PAGE and western or dot blotting of cell lysates using a gD-specific monoclonal antibody. A parental plaque (plaque #11) is selected and triple plaque purified. This recombinant baculovirus, designated D2Ac-11, is used for further glycoprotein expression.

**D. Preparation and Harvest of Sf9 Lysate.** Sf9 cells are grown in perfused suspension cultures in bioreactors (New Brunswick Scientific, Edison NJ) and infected at a cell density of $2 \times 10^6$ to $8 \times 10^6$ cells/ml with recombinant baculovirus at a multiplicity of infection of 0.1. Cultures are harvested at 44 hour postinfection (pi) by adjusting the baculovirus infected Sf9 cell suspension to pH 7.4 with either 0.1 N NaOH or 1.0 M EDTA prior to lysis by the addition of CHAPS detergent, to a final concentration of 60 mM.

**E. Purification of Native and Baculovirus Expressed Glycoproteins.** Native gD1 and gD2 are purified from detergent extracts of HSV-infected Vero cells, as previously described (25). In an analogous fashion, the baculovirus expressed gD2 glycoprotein is recovered from recombinant baculovirus-infected Sf9 insect cells. In brief, the pH of the infected Sf9 culture is adjusted to neutrality with 0.1 N NaOH or 1.0 M EDTA, and lysed by the addition of CHAPS (15-60 mM final concentration, depending on cell density) and/or other solubilizing agents. The detergent-solubilized glycoprotein is subjected to clarification using tangential flow ultrafiltration through 0.1 $\mu$m filters (Sarticon Inc., East Granby, CT) or similar processes followed by ion-exchange chromatography using DEAE Fast Flow resin (Pharmacia, Piscataway, NJ). Ion exchange flow through fractions are concentrated by tangential flow ultrafiltration using 20,000 molecular weight cut-off filters (Sarticon) and then resolved by immunoaffinity chromatography with solid-phase DL6 monoclonal antibody (12, 7; obtained from Drs. G.H. Cohen and R.J. Eisenberg) coupled to Activated CH-Sepharose (Pharmacia), following protocols provided by the manufacturer. Purified glycoprotein is eluted from the immunoaffinity matrix using buffered NaSCN (50 mM Tris pH 7.4, 0.5 M NaCl, 1.5 M NaSCN, 0.1% NP40), dialyzed against PBS, and then formulated into vaccine. Native gD1 only is treated with formalin (1:4000, 40 hours at $37^{o}$C). Aluminum phosphate ($A1PO_4$) is used as an adjuvant at 500 $\mu$g/dose. Glycoprotein dose concentration is confirmed directly by pyrochemiluminescent nitrogen analysis (Antek Inc., Houston, TX) and indirectly by dot blot analysis.

**F. SDS-PAGE and Western Blot Analysis.** Native gD1, gD2 and baculovirus expressed gD2 are subjected to SDS-PAGE and electrotransferred essentially as described by Symington (39). The nitrocellulose blots are incubated with the appropriate antibody and the antigen/antibody complex visualized with peroxidase labeled protein A and 4-chloronaphthol (Sigma Chemical Co., St. Louis MO). Antibodies used include a type-common rabbit polyclonal anti-gD, DL6 monoclonal antibody (McAb) which recognizes a sequential epitope of gD containing amino acid residues 272-279 (12, 7) and the monoclonal antibody 1D3 which recognizes a linear epitope in the region of amino acids 1-23 near the amino terminus of gD (28). Alternatively, nitrocellulose electroblots are treated with Aurodye (Amersham) to visualize total protein.

**G. Identity and Purity of bgD2.** The identity of the purified recombinant protein is confirmed by Western blot analysis using the gD specific monoclonal antibodies DL6 and 1D3 (N terminal specific), as well as anti-gD polyclonal serum. A comparative SDS-PAGE Western immunoblot of the three glycoproteins used in this study is presented in Figure 2A. Native gD1 forms a dimer after purification, which is not observed in either native or recombinant gD2. Recombinant gD2 is approximately the same molecular weight as native gD2.

The purity of bgD2 is confirmed by electroblotting an SDS-PAGE gel followed by silver staining (Figure 2B). Recombinant gD2 migrates as a single protein component with an apparent MW of 57,500. The direct correspondence between components visualized by antibody probes and those observed upon protein staining is consistent with glycoprotein purity in excess of 90%.

## II. Vaccine Administration and bgD2 Immunoreactivity

**A. Mice.** Female BALB/c VAF Plus (virus antibody free) mice were obtained from Charles River (Wilmington, MA) for use in immunogenicity and protection studies and are housed in sterilized micro-isolators.

**B. Formulation of gD and Immunization of Mice for In Vivo Studies.** Native gD1, gD2 and bgD2 fractions at concentrations between 20 and 140 $\mu$g/ml are mixed with $AlPO_4$ (2 mg/ml) at $4^{o}$ C overnight. The concentration of glycoprotein is then determined by either measuring total nitrogen content or by dot blot analysis. Samples for dot blot analysis are prepared in 96 well plates as two-fold dilutions in phosphate buffered saline (PBS) diluent. Purified, soluble glycoprotein is routinely used as a standard. Sample dilutions are transferred to a dot blot manifold (BRL, Gaithersburg, MD) and vacuum-absorbed onto the nitro-cellulose membrane. Each sample is washed 3X with 200 $\mu$g PBS, and the filter air dried prior to blocking for 45 minutes in 3% bovine serum albumin/Tris-buffered saline (BSA/TBS). Blots are then incubated for 1 hour with the selected antibody diluted in 1% BSA/TBS/0.05% Tween 20 (TTBS). In addition to antibodies used for Western blot analysis, DL11 (obtained from Drs. G.H. Cohen and R.J. Eisenberg), a non-sequential neutralizing McAb is employed (27, 29). Subsequently, antibody is removed by washing and the filter incubated with peroxidase-labeled protein A in 1% BSA/TTBS for 1 hour. The filter is then extensively washed and incubated with 4-chloronaphthol/$H_2O_2$ substrate in methanol. Formulated glycoprotein samples are analyzed in the same manner, except that the $AlPO_4$ is first removed from the filter by solubilization, in situ, with 1N HCl at $37^{o}$ C for 5 minutes. The glycoprotein/$AlPO_4$/PBS stock is then diluted to selected dose concentrations. The treatment groups (N = 16) are as follows: $AlPO_4$, 0.1 $\mu$g bgD2, 1.0 $\mu$g bgD2, 5.0 $\mu$g bgD2, 10.0 $\mu$g bgD2, 5.0 $\mu$g ngD2, 5.0 $\mu$g ngD1; uninoculated N = 6. Final glycoprotein concentrations are confirmed by dot blot analysis. Prior to immunization, the formulated glycoproteins are also probed by dot blot for the presence of neutralizing epitope DL11. All three glycoproteins contain this epitope.

Six-week-old mice are immunized with baculovirus-expressed gD2 preabsorbed to 500 $\mu$g of $AlPO_4$. Doses used are 0.1 $\mu$g, 1.0 $\mu$g, 5.0 $\mu$g, and 10.0 $\mu$g. Controls consist of mice immunized with 5.0$\mu$g of ngD1 or ngD2 or animals mock-immunized with $AlPO_4$ alone. All animals receive two subcutaneous immunizations in the scapular region at 3-week intervals.

**C. In Vitro Assays.** Two weeks following the second immunization, 5 mice of each dosing group are exsanguinated under anesthesia, and splenectomized for humoral and cellular analysis of immune responsiveness to vaccine.

(i) ELISA responses are evaluated to confirm in vivo equivalence of viral derived and baculovirus-expressed gD2. Ninety-six well plates (Corning, Corning, NY) are coated overnight with ngD1 or bgD2 at a concentration of 20 ng/well in 100 $\mu$l of 0.01M PBS. Plates are washed and incubated for 3 hours with 3% BSA at room temperature. Following BSA treatment, experimental sera are subjected to 2:fold dilutions across the plates in TBS + 0.1% Tween 20 and blocked for 1 hour at 37° C. Plates are washed 3X with PBS and then incubated with 100 $\mu$l of 1:500 dilution of horseradish peroxidase conjugated f(ab$_2$) goat-anti mouse IgG (Cappel, Organon Teknika, Durham, NC) in TBS. Plates are developed using ABTS substrate (Kirkegaard & Perry, Gaithersburg, MD) and quantitated at 405 nm using a Biotek ELISA plate reader (Winooski, VT).

Antibody responses, measured by ELISA, using bgD2 as in vitro antigen, indicate that all immunized mice are significantly responsive to vaccine (95% confidence interval) and that all vaccine treatments are significantly elevated relative to AlPO$_4$ control animals (P<0.001 by ANOVA). bgD2 immunized mice demonstrate generally dose-responsive ELISA titers with minima and maxima of activity recorded at 0.1 $\mu$g to 10.0 $\mu$g glycoprotein, respectively. At serum dilutions of greater than 1:160, responses of 0.1 $\mu$g bgD2 test sera for ngD1 antigen are significantly (P<0.05 by ANOVA) lower than all other immunized groups.

ELISA responses to ngD1 antigen are lower than those observed for bgD2. This most likely reflects a greater efficiency of binding of the recombinant relative to the native glycoprotein rather than significant differences in antibody responsiveness. However, ELISA responses mirror those of DL6 dot blots (data not shown) where McAb reactivity for baculovirus expressed glycoprotein is greater than that for native gD1. Anti-ngD1 ELISA activity for bgD2 immunized mice increased dose-dependently. Peak activity to ngD1 antigen is associated with ngD1 immunization. It is difficult to directly compare native glycoprotein responses with those of the baculovirus-expressed gD2 mice as native glycoprotein may contain traces of other HSV proteins, such as gB, which may contribute to the measured ELISA response. However, in this context, 5.0 $\mu$g ngD2, also potentially harboring trace contaminants, elicits a relatively low ELISA response against ngD1, comparable to 0.1 $\mu$g to 1.0 $\mu$g bgD2.

(ii) Neutralizing Antibody Assays. Neutralization assays are performed as previously described (21). Briefly, sera are heat inactivated at 56° C for 30 minutes prior to assay. Duplicate two-fold serial dilutions were mixed with 100 pfu of HSV1-NS or HSV2-186 in MEM containing 5% FCS and 10% guinea pig complement (Gibco, Grand Island, NY). Virus-serum mixtures are incubated for 1 hour at 37° C and then inoculated onto Vero cell monolayers grown in 96-well plates. Virus is absorbed for 1 hour (37° C) and then overlaid with 1% methylcellulose. Virus, medium and complement controls are included in each assay. Plates are incubated until >50 plaques are observed in virus control wells. Plates are then fixed in 10% formalin and plaques enumerated. Titers are defined as the reciprocal serum dilution demonstrating a mean plaque number less than 50% of that observed in virus controls.

Results are shown in Table 1. With the exception of a single animal immunized with 0.1 $\mu$g bgD2, all immunized mice demonstrated neutralizing antibody titers of 20 or greater against HSV1. Mean geometric mean titers to HSV1 (GMT1) for bgD2 mice generally increase in a dose-related fashion with minima and maxima at 0.1 $\mu$g and 10.0 $\mu$g, respectively. Mice treated with 5.0 $\mu$g bgD2 demonstrate a somewhat lower GMT1 (i.e., 183.9) than predicted by dose-response. This reflects a relatively low titer of 40 observed in a single animal. of interest are the enhanced GMT1 of bgD2 mice when compared with animals receiving ngD2. Indeed, 2.5-6.0 fold elevations of GMT1, relative to ngD2 immunization, occur at doses of 1.0 through 10.0 $\mu$ bgD2. Equivalent GMT1 are recorded for animals which receive either 10.0 $\mu$g bgD2 or 5.0 $\mu$g ngD1, demonstrating a good degree of cross-reactive neutralizing antibody activity for the recombinant preparation.

GMT2 are elevated in all vaccine treatment groups relative to HSV1 neutralizing titers. This is particularly evident for the 0.1 $\mu$g bgD2 group which increases by a factor of 24. ngD1 titers are essentially equivalent for HSV1 and HSV2 with GMT2/GMT1 of 1.5. Peak GMT2 is associated with 5.0 $\mu$g bgD2, attributable, in part, to a single animal displaying a titer of 10,240. A pattern of proportional cross-reactive response, as indicated by GMT2/GMT1, is noted for 5.0 $\mu$g bgD2 and 5.0 $\mu$g ngD2 where, in spite of higher titers recorded for the recombinant candidate, ratios of 13.9 are calculated for both groups.

TABLE 1

| Effect of bgD2 Immunization on Serum Neutralizing Antibody Titers Against HSV | | | | |
|---|---|---|---|---|
| Treatment | Dose (1g) | GMT-HSV1[a] | GMT-HSV2 | GMT2/GMT1 |
| AlPO$_4$ | | <20 | <20 | |
| bgD2 | 0.1 | 23.0 | 557.6 | 24.2 |
| bgD2 | 1.0 | 278.8 | 1115.1 | 4.0 |
| bgD2 | 5.0 | 183.9 | 2561.9 | 13.9 |
| bgD2 | 10.0 | 422.6 | 1254.6 | 3.0 |
| ngD2 | 5.0 | 69.7 | 970.8 | 13.9 |
| ngD1 | 5.0 | 422.6 | 640.5 | 1.5 |

**Note:**

[a] Geometric Mean Titers of neutralizing antibody responses against HSV1 (GMT1) and HSV2 (GMT2) were obtained from individual mice using a plaque reduction microneutralization assay method as described above.

(iii) Lymphoproliferative Assays. Spleen cells from immunized or AlPO$_4$-injected mice are plated at $2x10^5$ cells/well in 96-well flat bottomed plates in the presence of medium, Vero ($2.5x10^6$ PFU equivalents/well), ngD1 (10 ng/well), bgD2 (20 ng/well) or a gD1 peptide containing amino acid residues 241-260 (differing from gD2 at two residues; 39, 40). The medium used in lymphoproliferative assays consists of RPMI (Cellgro, Mediatech, Washington, DC) containing 10% fetal calf serum (Hyclone, Logan, UT), glutamine, HEPES, NEAA, pyruvate, $\beta$-mercaptoethanol and penicillin/streptomycin. After 5 days of incubation, cells are pulsed with $1\mu$Ci of [$^3$H] thymidine (Amersham, Arlington Heights, IL) for 6 hours and then are harvested for scintillation counting. Stimulation indices are calculated by dividing experimental CPM by CPM of medium controls.

Results are shown in Table 2. In vitro proliferative responses to whole viral antigens are essentially type-specific in terms of maximal T cell activation. Indeed, ngD1 immunized mice have significantly higher responses to HSV1 stimulus (P<0.01) than all other groups. Responses of bgD2-immunized mice to HSV2, and other stimuli, are characterized by inverse dose-response with peak activity seen at 0.1 $\mu$g of vaccine. ngD1-immune animals respond to ngD1 in vitro stimulation with a significantly enhanced uptake of $^3$H-thymidine relative to all other groups (P<0.04 by ANOVA). As noted for ELISA responses, the enhanced activity of ngD1-immune spleen cells may mirror the presence of traces of other HSV1 proteins capable of eliciting T cell blastogenesis. However, as in humoral responses, ngD2 immunization is not accompanied by any apparent elevation in response. A high degree of cross-reactive response between bgD2 and ngD1 is seen in cultures stimulated with D1 peptides aa241-261. This gD1 peptide differs from published gD2 sequence at two residues (#246 A≫P, #257 E≫D) and contains an immunodominant T$_h$ epitope (10).

## TABLE 2

## Lymphoproliferative Responses

## of gD-Immunized and Control Mice to HSV Antigens

In Vitro Stimulus (SI $\pm$ SE)[a]

| Vaccine | Dose[b] | HSV1 | HSV2 | ngD1 | bgD2 | aa241-261 |
|---|---|---|---|---|---|---|
| AlPO$_4$ | -- | 2.73+0.60 | 1.26+0.42 | 1.25+0.21 | 0.98+0.22 | 1.31+0.26 |
| bgD2 | 0.1 | 3.54+1.00 | 7.27+2.83 | 9.08+2.93 | 13.97+5.26 | 10.61+4.25 |
| bgD2 | 1.0 | 2.71+0.68 | 4.78+1.64 | 7.21+2.54 | 11.46+3.36 | 9.69+2.49 |
| bgD2 | 5.0 | 2.40+0.57 | 2.44+0.75 | 5.35+1.62 | 8.16+1.37 | 6.09+0.94 |
| bgD2 | 10.0 | 2.60+1.01 | 3.56+1.09 | 5.77+2.24 | 10.66+4.51 | 7.54+3.13 |
| ngD2 | 5.0 | 2.36+0.46 | 3.07+0.59 | 3.98+1.05 | 7.61+1.77 | 4.03+0.87 |
| ngD1 | 5.0 | 9.02+2.43 | 3.63+1.04 | 18.53+7.35 | 10.31+1.65 | 9.15+4.77 |

**Notes:**

[a] Lymphoproliferative assays were performed as described above. The mean and standard error of stimulation indices (SI) for 5 animals per group are presented. $SI = CPM_{experimental}/CPM_{medium\ control}$.

[b] Doses are in $\mu g$.

**D. Protection against HSV Challenge.** Two weeks following a second immunization, mice are inoculated subcutaneously (sc) with $1 \times 10^6$ pfu/0.03 ml of HSV2 (186) in the right rear footpad. Mice are monitored for HSV2-related disease for 30 days pi. Symptoms are evaluated on a daily basis and scored as follows: 0 = no symptoms; 1 = swelling or abrasion; 2 = weakness; 3 = partial paralysis of right hindlimb; 4 = full paralysis of right hindlimb; 5 = bilateral paralysis; 6 = death.

As shown in Table 3, all immunized groups of mice are significantly protected against lethal HSV2 challenge as manifested in reduced morbidity, a reduced frequency of paralytic infection, reduced mortalities (whereas 6 of 11 control mice succumb to infection, only a single immunized mouse of any group die; this animal received 5.0 $\mu g$ of ngD2 and had been paralyzed prior to its death), and increased frequencies of animals free from all observable signs of local or systemic infection. This protective immunity is apparent even at the low 0.1 $\mu g$ dose of baculovirus-expressed gD2, indicating a high degree of vaccine-mediated resistance to homotypic challenge.

TABLE 3

| Effect of bgD2 Immunization on Protection Against HSV2 Challenge | | | | | |
|---|---|---|---|---|---|
| Vaccine Treatment | Dose µg | Maximum Symptom + SE[a] | Frequency of Paralysis[b] | Frequency of Mortality | Frequency of Symptom Free |
| AlPO$_4$ | -- | 4.91±.37** | 11/11§ | 6/11¶ | 0/11§ |
| bgD2 | 0.1 | 1.27±.47 | 2/11 | 0/11 | 6/11 |
| bgD2 | 1.0 | 0.27±.27 | 1/11 | 0/11 | 10/11 |
| bgD2 | 5.0 | 0.45±.31 | 1/11 | 0/11 | 9/11 |
| bgD2 | 10.0 | 0.36±.24 | 0/11 | 0/11 | 9/11 |
| ngD2 | 5.0 | 0.27±.26 | 1/11 | 1/11 | 10/11 |
| ngD1 | 5.0 | 0.64±.32 | 1/11 | 0/11 | 10/11 |

**Notes:**

[a] Symptom scores were defined as 0 = no symptoms; 1 = local swelling/erythema/abrasion; 2 = weakness; 3 = partial paralysis of right hindlimb; 4 = complete paralysis of right hindlimb; 5 = bilateral paralysis; 6 = death.

b Frequency of paralysis includes animals demonstrating both partial and total impairment.

** P<0.01 by ANOVA.

§ P<0.01 by Fisher's Exact Test.

¶ P<0.04 by Fisher's Exact Test.

## III. Sequencing of bgD2 Glycoprotein

The nucleotide sequence of the glycoprotein D2 gene is determined by directly sequencing the viral DNA of HSV2 (strain 12) and D2Ac-11 (baculovirus recombinant). Overlapping fragments (see below) of the gD2 gene are amplified asymmetrically by the polymerase chain reaction (Perkin Elmer Gene Amp PCR Kit) to generate single strands and then sequenced by the dideoxy method with the U.S. Biochemical Sequenase kit Version 2.0.

The nucleotide sequence of the gD2 gene in the baculovirus recombinant D2Ac-11 matches the sequence of the parent gene from HSV2 (12). This sequence is also compared to a published strain, HSV2 strain G (Watson, R. Gene, 1983, 26:307-312).

The following are the base differences between the gD2 genes of strain G and D2Ac-11. Base number 268 is A of the ATG start site. Base number 1449 is G of the TAG stop site.

| Nucleotide | | | Amino Acid | |
|---|---|---|---|---|
| Base Number | D2Ac-11 | Strain G | D2Ac-11 | Strain G |
| 1000 | C | T | LEU | LEU |
| 1002 | C | A | | |
| 1325 | T | C | VAL | ALA |

These base differences are confirmed by sequencing both strands.

```
_____gD2_____

     235<_____>735

          <_____476

             480<_____>987

                  <_____800

          691<_____>1105

                    905<_____>1315

                         1081<_____>1480
```

## BIBLIOGRAPHY

**1. Blacklaws, B.A., Nash, A.A., and G. Darby.** 1987. Specifity of immune response of mice to herpes simplex virus glycoproteins B and D constitutively expressed on L cell lines. J. Gen. Virol. 68:1103-1114.

**2. Cabral, G.A., Fry, D., Marciano-Cabral, F., Lumpkin, C., Mercer, L., and D. Goperud.** 1983. A herpes virus antigen in premalignant and malignant biopsies and explants. Am. J. Obstet. Gynecol. 145:79-86.

**3. Cappel, R., Sprecher, S., DeCuyper, F., and J. DeBraekeleer. 1985.** Clinical efficacy of a herpes simplex virus subunit vaccine. J. Med. Virol. 16:137-145.

**4. Cohen, G.H., Katze, M., Hydrean-Stern, C., and R.J. Eisenberg.** 1978. Type-common CP-1 antigen of herpes simplex virus is associated with a 59000-molecular weight envelope glycoprotein. J. Virol. 27:172-181.

**5. Cohen, J., Charpilienne, A., Chilmonczyk, S., and M.K. Estes.** 1989. Nucleotide sequence of bovine rotavirus gene 1 and expression of the gene product in baculovirus. Virology. 171:131-140.

**6. Duffy (ed.), 1980.** Vaccine Preparation Techniques, Noyes Data Corporation, Park Ridge, New Jersey.

**7. Eisenberg, R.J., Long, D., Ponce DeLeon, M., Matthews, J.T., Spear, P.G., Gibson, M.G., Lasky, L.A., Berman, P., Golum, E., and G.H. Cohen.** 1985. Localization of epitopes of herpes simplex virus type 1 glycoprotein D. J. Virol. 53:634-644.

**8. Estes, M.K., Crawford, S.E., Penaranda, M.E., Petrie, B.L., Burns, J.W., Chan, W., Ericson, B., Smith, G.E., and M.D. Summers.** 1987. Synthesis and immunogenicity of the rotavirus major capsid antigen using a baculovirus expression system. J. Virol. 61:1488-1494.

**9. Frenkel, L.M., Dillon, M., Garratty, E., Bryson, Y., Hackell, J., Cawein, A., Abramovitz, A., and Mishkin, E. 1990. Abstr. A randomized** double blind, placebo-controlled phase I trial of a herpes simplex virus purified glycoprotein (gD1) vaccine. The 30th ICAAC meeting, 1990.

**10. Fuller, A.O. and P.G. Spear.** 1987. Anti-glycoprotein D antibodies that permit absorption but block infection by herpes simplex virus 1 prevent virion fusion at the cell surface. Proc. Natl. Acad. Sci. 84:5454-5458.

**11. Heber-Katz, E., Hollosi, M., Dietzschold, B., Hudecz, F., and G.P. Fasman.** 1985. The T cell response to the glycoprotein D of herpes simplex virus: the significance of antigen conformation. J. Immunol. 135:1385-1390.

**12. Highlander, S.L., Sutherland, S.L., Gage, P.J., Johnson, D.C., Levine, M., and J.C. Glorioso.** 1987. Neutralizing monoclonal antibodies specific for herpes simplex virus glycoprotein D inhibit virus penetration. J. Virol. 61:3356-3364.

**13. Jarvis, D.L. and M.D. Summers.** Glycosylation and secretion of human tissue plasminogen activator in recombinant baculovirus-infected insect cells. Molecular and Cellular Biology. 9:214-223.

**14. Krishna, S., Blacklaws, B.A., Overton, H.A., Bishop, D.H.L., and A.A. Nash.** 1989. Expression of glycoprotein D of herpes simplex virus type 1 in a recombinant baculovirus: Protective responses and T cell recognition of the recombinant infected cells. J. Gen. Virol. 70:1805-1814.

**15. Lanford, R.E., Luckow, V., Kennedy, R.C., Dreesman, G.R., Notvall, L., and M.D. Summers.** 1989. Expression and characterization of hepatitis B virus surface antigen polypeptides in insect cells with a baculovirus expression system. J. Virol. 63:1549-1557.

**16. Lasky, L.A. and D.J. Dowbenks.** 1984. DNA sequence analysis of the type common glycoprotein D genes of herpes simplex virus types 1 and 2. DNA 3:23-29.

**17. Luchow, V.A. and M.D. Summers.** 1988. Trands in the development of baculovirus expression vectors. Bio/Technology 6:47-55

**18. Luckow, V.A. and M.D. Summers.** 1989. High level expression of nonfused foreign genes with Autographa californica nuclear polyhedrosis virus expression vectors. Virology. 170:31-39.

**19. Maeda, S., Kawai, T., Obinata, M., Fugivara, H., Horiuchi, T., Saeki, Y, Sato, Y., Furusawa, M.** 1985. Production of human $\alpha$-interferon in silkworm using a baculovirus vector. Nature 315:592-594.

**20. Maiorella, B., Inlow, D., Shauger, A., and D. Harano.** 1988. Large scale insect cell culture for recombinant protein production. BioTechnology. 6:1406-1410.

**21. Maniatis, T., Fritsch, E.F., and J. Sambrook.** 1982. Molecular Cloning. A Laboratory Manual, Cold Spring Harbor Laboratory. Cold Spring Harbor, NY.

**22. Martin, S., Moss, B., Berman, P.W., Lasky, L.A., and B.T. Rouse.** 1987. Mechanisms of antiviral immunity induced by a vaccinia virus recombinant expressing herpes simplex virus type 1 glycoprotein D: cytotoxic T cells. J. Virol. 61:726-734.

**23. Matsura, Y., Possee, R.D., Overton, H.A. and Bishop, D.H.L.** 1987. Baculovirus expression vectors: the requirements for high level expression of proteins, including glycoproteins. J. Gen. Virol. 68:1233-1250.

**24. Melnick, J.L., Adam, E., and W. Rawls.** 1974. The causative role of herpes virus type 2 in cervical cancer. Cancer 34:1355-1385.

**25. Mishkin, E.M., Fahey, J.R., Kino, Y., Klein, R.J., Abramovitz, A.S., and S.J. Mento.** 1991. Native herpes simplex virus glycoprotein D vaccine: immunogenicity and protection in animal models. Vaccine. 9:147-153.

**26. Mosmann, T.R. and R.L. Coffman.** 1989. Heterogeneity of cytokine secretion patterns and functions of helper T cells. Adv. Immunol. 46:111-147.

**27. Muggeridge, M.L., Wu, T.T., Johnson, D.C., Glorioso, J.C., Eisenberg, R.J., and G.H. Cohen.** 1990. Antigenic and functional analysis of a neutralization site of HSV-1 glycoprotein D. Virology. 174:375-387.

**28. Muggeridge, M.L., Roberts, S.R., Isola, V.J., Cohen, G.H. and R.J. Eisenberg.** 1990. Herpes simplex virus. pp. 459 = 481. In M.H.V. van Regenmortel and A.R. Neurath (ed.) Immunochemistry of viruses. II. The basis for serodiagnosis and vaccines. Elsevier, New York.

**29. Muggeridge, M.I., Isola, V.J., Byrn, R.A., Tucker, T.J., Minson, A.C., Glorioso, J.C., Cohen, G.H., and R.J. Eisenberg.** 1988. Antigenic analysis of a major neutralization site of herpes simplex virus glycoprotein D, using deletion mutants and monoclonal antibody-resistant mutants. J. Virol. 62:3274-3280.

**30. Nahmias, A.J. and R.M. Coleman.** 1984. The significance of herpes simplex virus infections in humans. pp. 92-102. In B.T. Roouse and C. Lopez (ed.) Immunobiology of herpes simplex virus infection, CRC Press, Boca Raton.

**31. Norrild, B., Shore, S.L., and A.J. Nahmias.** 1979. Herpes simplex virus glycoproteins: participation of individual herpes simplex virus type 1 glycoprotein antigens in immunocytolysis and their correlation with previously identified glycopeptides. J. Virol. 32:741-748.

**32. Paoletti, E., Lipsinskas, B.R., Samsonoff, C., Mercer, S., and D. Panicali.** 1984. Construction of live vaccines using genetically engineered poxviruses: biological activity of vaccinia virus recombinants expressing the hepatitis virus surface antigen and the herpes simplex virus glycoprotein D. Proc. Natl. Acad. Sci. 81:193-197.

**33. Reid-Sanden, F.L., Sumner, J.W., Smith, J.S., Fekadu, M., Shaddock, J.H., and W.J. Bellini.** 1990. Rabies diagnostic reagents prepared from a rabies N gene recombinant expressed in baculovirus. J. Clin. Microbiol. 28:858-863.

**34. Schmaljohn, C.S., Parker, M.D., Ennis, W.H., Dalrymple, J.M., Collett, M.S., Suzich, J.A., and A.L. Schmaljohn.** 1989. Baculovirus expression of the M genome segment of rift valley fever virus and examination of antigenic and immunogenic properties of the expressed proteins. Virology. 170:184-192.

**34. Schrier, R.D., Pizer, L.I., and J.W. Moorhead.** 1983. Type-specific delayed type hypersensitivity and protective immunity induced by isolated herpes simplex virus glycoprotein. J. Immunol. 130:1413-1418.

**35. Smith, G.E., Summers, M.D., and Fraser, M.J.** 1983. Production of human beta-interferon in insect cells infected with a baculovirus expression vector. Mol. Cell. Biol. 3:2156-2165.

**36. Stanberry, L.R., Burke, L.R., and M.G. Myers.** 1988. Herpes simplex virus glycoprotein treatment of recurrent genital herpes. J. Infect. Dis. 157:156-163.

**37. Stanberry, L.R., Bernstein, D.I., Burke, R.L., Pachl, C., and M.G. Myers.** 1987. Vaccination with recombinant herpes simplex virus glycoproteins: Protection against initial and recurrent genital herpes. J.

Infect. Dis. 155:914-920.

**38. Summers, M.D., and G.E. Smith.** 1987. A Manual of Methods for Baculovirus Vectors and Insect Cell Culture Procedures. Texas Agricultural Experiment Station Bulletin No. 1555.

**39. Symington, J.** 1984. Electrophoretic transfer of proteins from two-dimensional gels to sheets and their detection, pp. 127-168. In J.E. Celis and R. Bravo (ed.), Two Dimensional Gel Electrophoresis of Proteins: Methods and Applications. Academic Press, NY.

**40. Torseth, J.W., Cohen, G.H., Eisenberg, R.J., Berman, P.W., Lasky, L.A., Cerini, C.P., Heilman, C.J., Kerwar, S., and T.C. Merigan.** 1987. Native and recombinant herpes simplex virus type 1 envelope proteins induce human immune T-lymphocyte responses. J. Virol. 61:1532-1539.

**41. Valenzuela, P., Coit, D., Medina-Selby, M.A., Kuo, C.H., Van Nest, G., Burke, L.R., Bull, P., Ardea, M.S., and P.V. Graves.** 1983. Antigen engineering in yeast: Synthesis and assembly of hybrid hepatitis B surface antigen-herpes simplex 1 gD particles. BioTechnology. 3:323-326.

**42. Wather, M.W., Brideau, R.J., and D.R. Thomsen.** 1989. Immunization of cotton rats with the human respiratory syncitial virus F glycoprotein produced using a baculovirus vector. J. Infect. Dis. 159:255-264.

**43. Watson, R.J.** 1983. DNA sequences of the herpes simplex virus type 2 glycoprotein D gene. Gene. 26:307-312.

**44. Watson, R.J., Weis, J.H., Salstrom, J.S., and L.W. Enquist.** 1982. Herpes simplex virus type 1 glycoprotein D gene: nucleotide sequence and expression in Escherichia coli. Science. 218:381.

**45. Wells, D.E., Vugler, L.G., and W.J. Britt.** 1990. Structural and immunological characterization of human cytomegalovirus gp55-116(gB) expressed in insect cells. J. Gen. Virol. 71:873-880.

**46. Wilcox, W.C., Lond, D., Sodora, D.L., Eisenberg, R.J., and G.H. Cohen.** 1988. The contribution of cysteine residues to antigenicity and extent of processing of herpes simplex virus type 1 glycoprotein D. J. Virol. 62:1941-1947.

**47. Williams, D.A., Chase, M.W. 1967.** Methods on Immunology and Immunochemistry. Volume 1. Academic Press, New York, pp. 197-237.

**48. Zarling, J.M., Moran, P.A., Lasky, L.A., and B. Moss.** 1986. Herpes simplex virus (HSV) specific human T-cell clones recognize HSV glycoprotein D expressed by recombinant vaccinia virus. J. Virol. 59:506-509.

## Claims

1. A method for recombinant production of HSV gD2 comprising transfecting an insect cell with baculovirus DNA incorporating a gene encoding HSV gD2, and culturing the cells under conditions that permit expression of the gene.

2. The method of Claim 1 wherein the HSV gD2 gene is under the control of the baculovirus polyhedrin promoter.

3. The method of Claim 2 wherein the baculovirus is Autographa californica.

4. The method of Claim 1 wherein the HSV gD2 is recovered and purified.

5. A substantially pure HSV gD2, obtained by the method of Claim 4.

6. A vaccine composition for the prevention and treatment of HSV infection comprising an effective amount of the gD2 of Claim 5, in combination with a pharmaceutically acceptable carrier.

7. A DNA construct comprising a baculovirus polyhedrin promoter operatively linked to a gene encoding HSV gD2.

8. A vector comprising the construct of Claim 7.

9. The vector of Claim 8 which is p941gD2C.

10. An insect cell containing the vector of Claim 8.

11. The insect cell of Claim 10 wherein the vector is p941gD2C.

12. A recombinant baculovirus containing and capable of expressing a gene encoding HSVgD2.

FIG.1A

FIG.1B

FIG. 2A

FIG. 2B

FIG.3A

OD$_{405}$ Units

-Log$_{10}$ Dilution

FIG. 3B

European Patent
Office

EUROPEAN SEARCH REPORT

Application Number

EP  92 11 3634
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X,D | GENE<br>vol. 26, no. 2/3, 1983, BRUSSELS BELGIUM<br>pages 307 - 312<br>WATSON, R.J. 'DNA sequence of the Herpes<br>Simplex Virus type 2 glycoprotein D gene'<br>* the whole document * | 5 | C12N15/38<br>C12N15/86<br>C12N5/10<br>A61K39/245<br>C07K13/00 |
| Y | | 1-12 | |
| X | HERPES SIMPLEX VIRUS VACCINE WORKSHOP 31<br>July 1989, BETHESDA, USA<br>& REVIEWS OF INFECTIOUS DISEASES<br>vol. 13, no. S 11, 1991, CHICAGO, USA<br>pages S924 - S934<br>AURELIAN, L. ET AL. 'Immune responses to<br>Herpes Simplex Virus in Guinea pigs<br>(footpad model) and mice immunized with<br>Vaccinia Virus recombinants containing<br>Herpes Simplex Virus glycoprotein D' | 5 | |
| Y | * the whole document * | 1-12 | |
| Y | JOURNAL OF GENERAL VIROLOGY<br>vol. 70, no. 7, 1989, UK<br>pages 1805 - 1814<br>KRISHNA, S. ET AL. 'Expression of<br>glycoprotein D of Herpes Simplex Virus<br>type 1 in a recombinant Baculovirus :<br>protective responses and T cell<br>recognition of the recombinant-infected<br>cell extracts'<br>* the whole document * | 1-12 | **TECHNICAL FIELDS SEARCHED (Int. Cl.5 )**<br><br>C07K<br>C12N |
| D,Y | VIROLOGY<br>vol. 170, 1989,<br>pages 31 - 39<br>LUCKOW, V.A. & SUMMERS, M.D. 'High level<br>expression of nonfused foreign genes with<br>Autographa californica Nuclear<br>Polyhedrosis Virus expression vectors'<br>* the whole document * | 1-12 | |

The present search report has been drawn up for all claims

| Place of search | Date of completion of the search | Examiner |
|---|---|---|
| THE HAGUE | 04 JANUARY 1993 | CHAMBONNET F.J. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)

European Patent Office

EUROPEAN SEARCH REPORT

Application Number

EP    92 11 3634
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | BIOTECHNOLOGY vol. 6, January 1988, NEW YORK US pages 47 - 55 LUCKOW, V.A. & SUMMERS, M.D. 'Trends in the development of baculovirus expression vectors' * the whole document * | 1-12 | |
| | ----- | | |

TECHNICAL FIELDS SEARCHED (Int. Cl.5 )

The present search report has been drawn up for all claims

| Place of search | Date of completion of the search | Examiner |
|---|---|---|
| THE HAGUE | 04 JANUARY 1993 | CHAMBONNET F.J. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P0401)